# EUROPEAN PATENT SPECIFICATION

(11) **EP 1 121 053 B1**
(45) Date of publication and mention of the grant of the patent: **13.04.2005**
(21) Application number: 99953102.3
(22) Date of filing: 07.10.1999
(51) Int. Cl.: A61B 5/117

(54) **PROTECTIVE ENCLOSURE FOR A FINGERPRINT SENSOR**
SCHUTZGEHÄUSE FÜR EINEN FINGERABDRUCKSENSOR
ENCEINTE DE PROTECTION POUR UN DISPOSITIF DE DETECTION D'EMPREINTES DIGITALES

(30) Priority: 12.10.1998 US 169894
(43) Date of publication of application: 08.08.2001
(73) Proprietor: STMicroelectronics N.V., 1215 Geneve 15 (CH)
(72) Inventor: O'GORMAN, Lawrence, Madison, NJ 07940 (US); MILLER, Wayne, H., Los Altos, CA 94022 (US)
(74) Representative: Driver, Virginia Rozanne
(86) International application number: PCT/US1999/023473
(87) International publication number: WO 2000/021439

(56) References cited:
- EP-A- 0 813 164
- GB-A- 1 267 996
- US-A- 3 804 524
- US-A- 3 848 184
- US-A- 5 177 802

## Description

The present invention relates to enclosures for sensor devices, and more particularly to a protective enclosure which aligns an object placed on a biometric sensor.

Biometric-oriented personal identification techniques are becoming increasingly important in protecting personal property, such as laptop computers and cellular phones, preventing credit card and calling card fraud, limiting access to security areas, computers and information, and ensuring security for electronic commerce. Biometric identification techniques use physical traits, measurements and characteristics specific to an individual. These characteristics include, but are not limited to, voice prints, hand prints, fingerprints, retina patterns, and signatures. Typically, biometric identification and verification techniques compare an individual's stored biometric data (the enrolled data) against newly obtained biometric data when the individual desires use of a protected item, access to a protected area or access to protected information. Due to its inherent nature, biometric data has the advantage of always being available for user identification and verification.

The fingerprint biometric is one of the most widely used and researched biometric identification techniques. Existing technology allows the relevant features of a fingerprint to be represented in a few hundred bytes of data. Furthermore, the computer hardware required for recording and comparing fingerprint data can be centralized and accessed through a telecommunications network, centralized databases and processing hardware, with the result that costs may be amortized across many more transactions than would be the case for distributed processing.

British Patent Number GB 1,267,996 discloses a system for identifying an individual by forming data sets containing the dimensions of an individual's fingers.

Document US-A-3 804 524 describes an apparatus for directing and positioning a finger for print identification comprising the features of the preamble of claim 1.

There are, however, disadvantages to biometric identification and verification. For instance, biometric sensors are susceptible to damage from impacts. Also, solid-state biometric sensors are susceptible to damage from electrostatic discharge. In fingerprint identification, the sensor may be particularly susceptible to electrostatic discharge, because the user touches the sensor during the sensing operation.

There are also problems associated with acquiring an accurate image of the fingerprint when an individual desires to access a protected item. In a typical enrollment procedure, the user centers the core of the fingerprint on the sensor, because the core provides desirable identification characteristics. Due to the relatively small size of most fingerprint sensors, often as small as 0.6 inches x 0.6 inches (150 mm x 150 mm), little, if any, of the rest of the fingerprint is sensed by the sensor. During an access procedure, users instinctively place their finger tips on the sensor. When a user places a portion of a fingerprint on the sensor which does not overlap the enrolled image, access will be denied due to finger placement error.

Accordingly, a device is needed for protecting the sensor from impacts, from electrostatic discharge and from other potentially harmful foreign materials, such as dust, dirt, sunlight and liquids. There is also a need for 8 device and method for aligning a finger on the sensor to ensure overlap with the enrolled image.

### SUMMARY OF THE INVENTION

The present invention increases accuracy and reliability of biometric identification and verification techniques while protecting a sensor from harmful impacts and electrostatic discharge. Such protection and increased accuracy and reliability an achieved by providing an enclosure comprising a movable access piece made from a conductive material. When the access piece is positioned to reveal the sensor, the walls of the enclosure, together with the access piece, cause the user to properly align a finger with the sensor. Protection from electrostatic discharge is achieved by grounding the access piece, which the user must move to reveal the sensor. When the sensor is not in use, the access piece is closed, protecting the sensor from harmful impacts.

The present invention encloses a biometric sensor for sensing fingerprints. The sensor is mounted relative to an access piece, and the access piece is positionable between a closed position and at least one open position, preferably slidable, to reveal the sensor. When the access piece is in the closed position, the sensor is completely enclosed. To access the sensor, the user slides the access piece to an open position using the finger he wishes to place on the sensor. When the access piece reaches the fully opened position the desired portion of the fingerprint is aligned with the sensor. The access piece is made from a conductive material, and it is electrically grounded. Since the user must touch the access piece to reveal the sensor, the user is electrostatically discharged before accessing the sensor.

The present invention is defined in the appended claims. All configurations which do not fall within the scope of these claims are of explanatory nature only.

### BRIEF DESCRIPTION OF THE FIGURES

For a better understanding of the present invention, reference may be had to the following description of exemplary configurations thereof, considered in conjunction with the accompanying drawings, in which:
FIG. 1A shows one view of an examplary enclosure;
FIG. 1B shows a cross-sectional view of the enclosure of FIG. 1A;
FIG. 1C shows another cross-sectional view of the enclosure of FIG. 1A;
FIG. 1D shows a top view of the enclosure of FIG.1A;
FIG. 1E shows another view of an exemplary enclosure;
FIG. 2 shows a side view of an exemplary enclosure;
FIG. 3 shows a top view of an exemplary enclosure;
FIG. 4A and 4B show a side view and a perspective view of an exemplary enclosure;
FIG. 5A, 5B, 5C and 50 show a top view of an exemplary enclosure with an access piece positionable at a pluratity of positions;
FIG. 6 shows a fingerprint image;
FIG. 7 shows a flow chart illustrating one exemplary method of operating a sensor and,
FIG. 8A and 8B show another exemplary configuration.

### DETAILED DESCRIPTION OF THE DRAWINGS

The present invention provides an apparatus for enclosing a biometric sensor. An enclosure according to the invention protects the sensor from harmful impacts, from electrostatic discharges (ESDs) and from other harmful material and electromagnetic energy. In the preferred embodiment, the enclosure protects a biometric sensor used for sensing fingerprints, and the enclosure is configured to cause a user to align the fingerprint core with the sensor during an access procedure. The enclosure of the invention is also used during enrollment. A method is provided for enrolling and reconstructing a fingerprint image which increases the likelihood of image overlap during an access procedure is described. Also, it is described an apparatus for indicating to the user when a fingerprint image of adequate quality is captured.

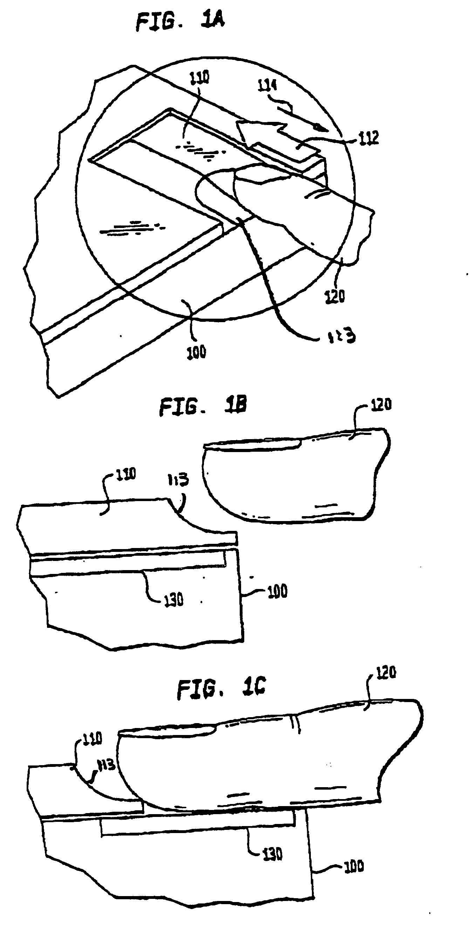

The preferred embodiment of the enclosure according to the invention is shown in FIG. 1A. The enclosure 100 comprises an access piece 110 which is shown in the closed position. The access piece 110 is a sliding door, and is movable in the direction of the arrows 112 and 114. A cross-section of the enclosure 100 with the access piece 110 in a closed position is shown in FIG. 1B. A sensor 130 is mounted in the enclosure 100 such that the closed access piece 110 covers the sensor 130, thereby protecting it from impacts. An exemplary embodiment of a fingerprint sensor device 130 that can be used in conjunction. with the present invention is explained in U.S Patent Application for a "Capacitive Fingerprint Sensor With Adjustable Gain," filed May 13, 1997, which is now U.S. Patent No. 6,049,620, that describes a method and an apparatus for detecting a fingerprint from a finger surface. The apparatus includes a planar array of capacitive sense elements disposed on a substrate. It also includes an insulating and receiving surface disposed over the array of sense elements, which is adapted to receive a finger so that a sense element and a portion of the finger surface located thereabove create a measurable change in capacitance. The capacitance is measured by first pre-charging each sense element, and then using a known current source to remove a fixed amount of charge from each capacitor plate. After a fingerprint is acquired, the quality of the fingerprint is evaluated, and if necessary, a gain parameter for the sense elements is iteratively adjusted until a satisfactory fingerprint is acquired.

Operation of the enclosure 100 is described with reference to FIG. 1A. FIG. 1B and FIG. 1C. A user accesses the sensor 130 by placing a finger 120 on the access piece 110 and moving it in the direction of the arrow 112. In this position, the sensor 130 is fully revealed, as shown in FIG. 1C, and the finger 120 has access to the sensor 130. The finger 120 will then be disposed on the sensor 130 in a proper position and the sensing operation may proceed. A spring (not shown) attaches the access piece 110 to the enclosure 100 such that the access piece 110 closes (is returned to the closed position) when the user releases the access piece 110.

To overcome the hazards of ESD in the sensor 130, especially during an access procedure, the access piece 110 is made of a conductive material and is electrically grounded. When a user touches the access piece 110 to access the sensor 130, the user is grounded through the access piece 110. Because The finger 120 must continue to apply pressure to the access piece 110 to overcome the force of the spring, the finger 120 remains grounded throughout the sensing operation. Once the user releases the access piece 110, it automatically closes, thereby enclosing the sensor 130.

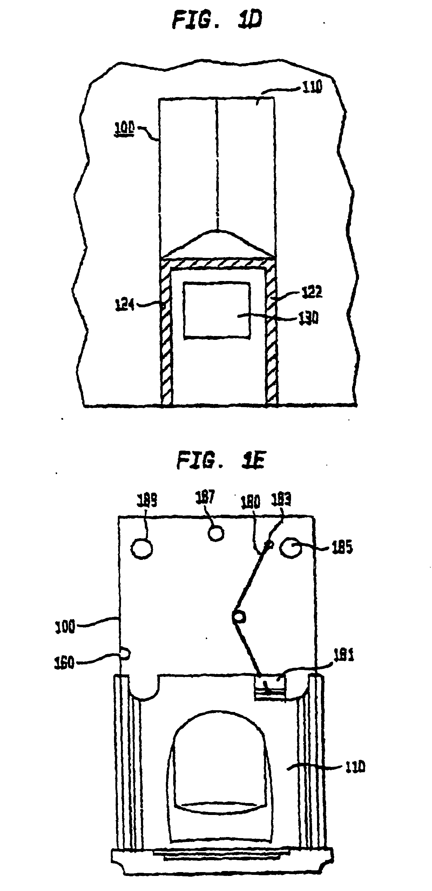

One exemplary spring configuration is shown in FIG. 1E. The spring 180 is a coil spring with elongated ends, each end having a hook. At one end, the spring 180 is hooked to a coupling protrusion 181 on the access piece 110. The other end is hooked to the enclosure 100 at an aperture 183. When the spring 180 is relaxed (that is, not under tension), the access piece 110 is closed. To open the access piece 110, the user must overcome the force of the spring 180. Upou release, the spring 180 returns the access piece 110 to the closed position.

As sbown in FIG. 1E, the enclosure further comprises means for mechanically fastening the enclosure 100 to some other device, such as a laptop computer. In the illustrated embodiment, the fastening means include a locating pin 187 and fastening toles 185 and 189. The locating pin 187 fits in a corresponding hole in the device of interest to locale the enclosure 100 in the desired position. Fastening holes 185 and 189 are configured to accept a corresponding fastening means, such as a screw.

A switch 160 attached to the enclosure 100 is also provided. Preferably the switch 160 is operable to switch power to the sensor on and off. The switch 160 is positioned relative to the access piece 110 so that the access piece 110 engages the switch when the user slides the access piece 110 to access the sensor (not shown). When the user releases the access piece 110, the spring 180 causes the access piece 110 to return to the closed position. After the movement to the closed position, the access piece 110 disengages the switch 160 thereby turning off power to the sensor (not shown).

It is another advantage of this enclosure 100 that the access piece 110 is configured to stop in a position which aligns the finger 120 with the sensor 130. Referring to the cross-section of the access piece shown in FIG. 1B, the access piece 110 is shaped to form a fingertip contour 113. As a user approaches the enclosure 100 to access the sensor 130, the user intuitively touches the access piece 110 in this contoured area 113 with the finger tip, because the fingertip naturally fits into the area 113. As shown in FIG. 1C, when the access piece 110 is moved to an open position with the fingertip placed in the contoured area 113, the top of the finger 120 extends beyond the sensor 130 and the fingerprint core is aligned with the sensor 130.

Lateral alignment of the finger 120 on the sensor 130 is shown with reference to FIG. 1D. The enclosure 100 comprises guides 122 and 124 spaced apart by a predetermined width, preferably the width of the finger 120. In the enclosure 100, the guides 122 and 124 are molded plastic walls. To accommodate fingers of various sizes, the walls may also be slanted inwardly from top to bottom; that is, toward the sensor. When the user places the finger 120 on the sensor 130, the guides laterally align the finger 120 on the sensor 130. The alignment provided by the access piece 110 in the open position and by the guides 122 and 124 enhances accuracy and reliability in acquiring the fingerprint image by minimizing finger placement error.

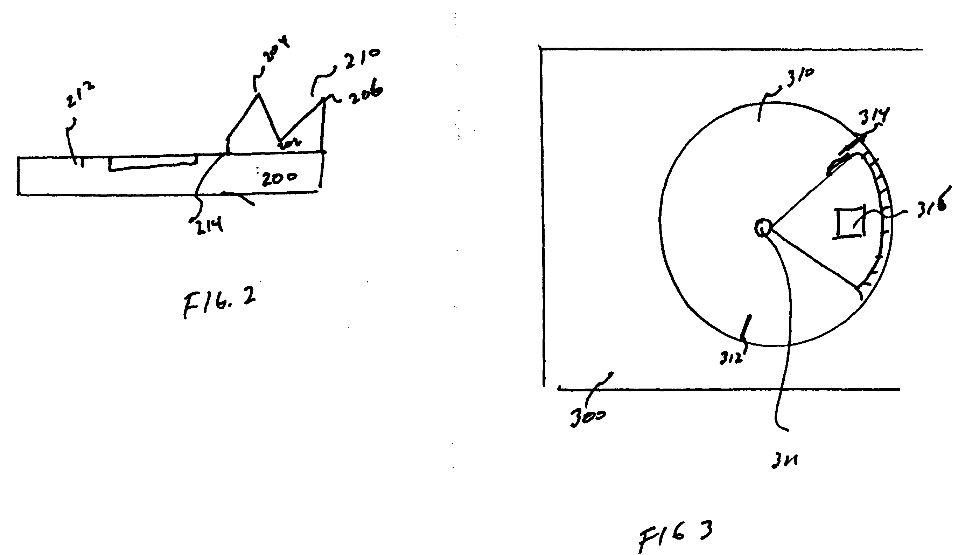

Of course, the access piece may be configured in various ways to protect sensors designed for various uses. For instance, with reference to FIG. 2, a side view of an enclosure 200 comprising a hinged 202, 204 and 206 access piece 210 is shown. The access piece 210 is made from a conductive material, and it is positionable at a closed position 212 and an open position 214. In the closed position 212, a sensor 220 is covered, protecting it 220 from impacts. To move the access piece 210, the user pushes the piece 210 with his finger to the open position 214. The same previously described alignment and grounding features may be provided.

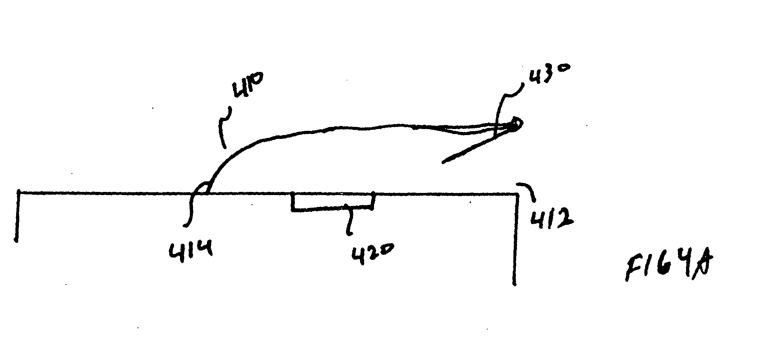

The top view of another configuration is shown in FIG. 3. The enclosure 300 comprises a rotatable access piece 310 which is positionable at a closed position 312 and at an open position 314. The user operates the access piece 310 by rotating it with his finger about a pivot 311 to the open position 314. The sensor 316 is revealed when the access piece 310 is at the open position 314. The access piece 310 is electrically conductive to ground, and is configured to return to the closed position when the user releases the access piece 310.

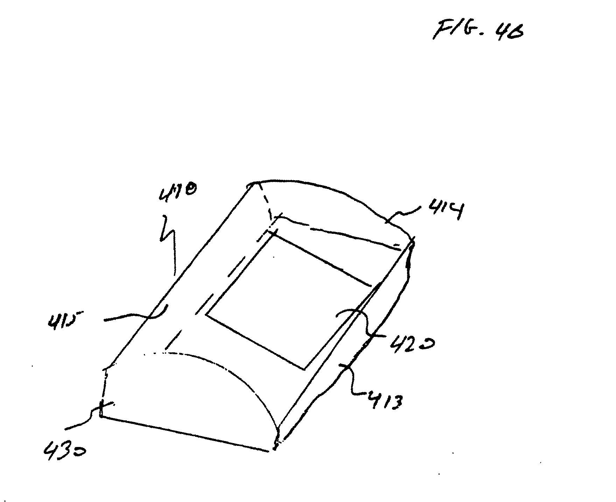

Still another configuration is shown in FIG. 4A. An enclosure 400 comprises a housing 410 with an access end 412 and a closed end 414. The housing 410 protects a sensor 420 from impacts when the sensor 420 is not in use. An access piece 430 covers the access end 412. The access piece 430 is made from a conductive material and is swingable between a closed position (not shown) and an open position. The user accesses the sensor 420 by pushing on the access piece 430 with his finger. The access piece 430 is grounded, thereby protecting the sensor 420 from ESD. Preferably, the housing 410 is shaped such that a finger placed within the housing is laterally aligned with the sensor 420. In this embodiment, the closed end 414 of the housing 410 acts as a stop, causing the user to align his finger with the sensor 420 such that the core of the fingerprint is on the sensor 420.

A perspective of this configuration is shown in FIG. 4B. Walls 413 and 415 laterally constrain the finger (not shown) such that the finger is laterally aligned on the sensor 420. The closed end 414 acts as a constraint causing the fingerprint core to locate on the sensor 414. The access piece 430 is grounded to protect the sensor 420 from electrostatic discharge.

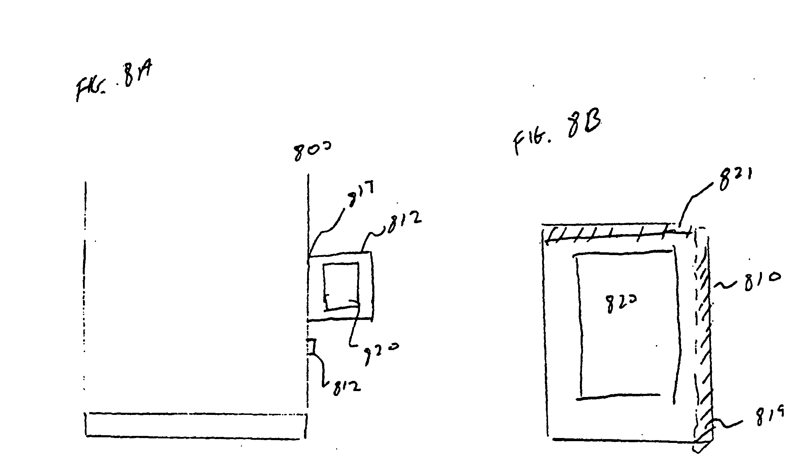

In still another configuration, the sensor is mounted in a slidable unit. As shown in FIG. 8A and FIG. 8B, the enclosure 800 comprises a sliding unit 810. In the closed position, the sliding unit 810 resides within the enclosure 800 and the sensor 820 is protected. An access piece 812, which is a button in this configuration, is operable to cause the sliding unit 810 to slide out of the enclosure 800. The means for sliding the sliding unit 810 into and out of the enclosure 800 may be a spring or motor. As in the prior configurations, the button 812 is electrically conductive to a ground. The user is grounded when he presses the button 812 to release the sliding unit 810. An enclosure edge 817 constrains the finger in one direction and sliding unit edges 819 and 821 constrain the finger in a second and third direction.

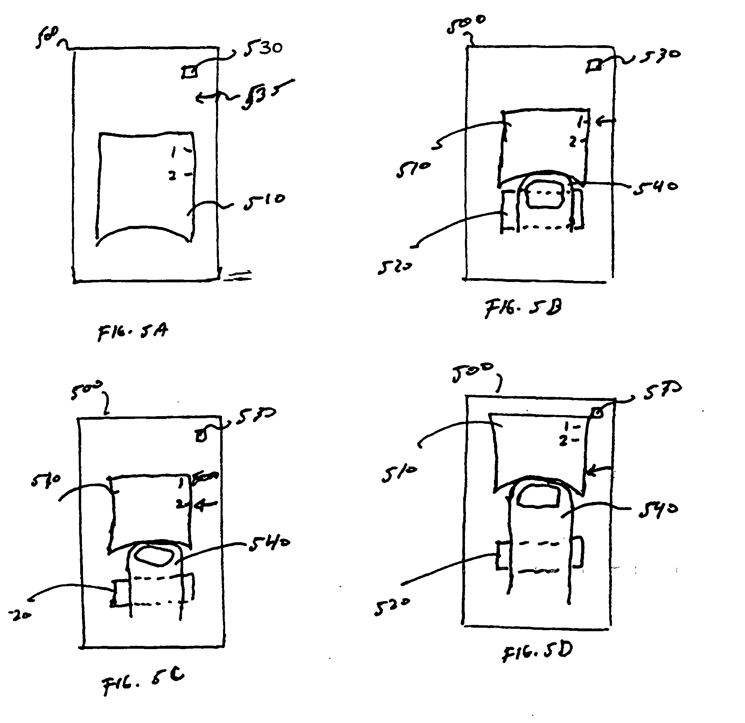

An enclosure is also described with an access piece positionable at a plurality of positions. Referring to FIG. 5A, an enclosure 500 is shown with an access piece 510 in a closed position, completely covering the sensor (not shown). The enclosure also comprises a stopper 530 which is operable to stop the access piece 510 at an open position. An arrow 535 marks a position on the enclosure 500, and a "1" and a "2," or other such alignment marks, mark two positions on the access piece 510. By aligning the access piece 510 markers "1" or "2" with the arrow 535, the access piece 510, in this case a sliding door, is positionable at multiple predetermined positions.

This multiple position capability enables capture of different portions of the fingerprint during enrollment. Enrollment is the procedure by which a fingerprint image is captured and stored as computer accessible data. In FIG. 5B, the enclosure 500 is shown with the access piece 510 positioned at position "1." Only the tip of the finger 540 extends beyond the sensor 520, and the finger 540 and sensor 520 are positioned such that the top of the fingerprint image may be captured. FIG. 5C shows the relative positions of the sensor 520, access piece 510 and finger 540 when the access piece 510 is at position "2." The finger 540 is positioned such that the fingerprint core is centered on the sensor 520, permitting capture of this portion of the fingerprint. In FIG. 5D, the access piece 510 is pushed to the stopper 530, and the finger 540 and sensor 520 are positioned such that an image of the bottom of the fingerprint may be captured.

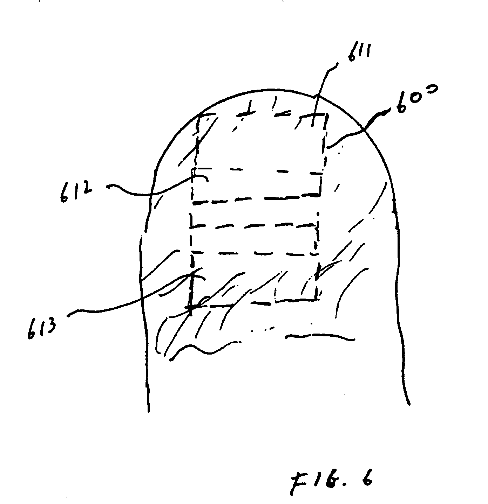

This procedure enables enrollment and reconstruction of a fingerprint image which comprises the combination of the images captured in position "1," position "2," and at the stop 530 position. This reconstructed image is known as a virtual image. This virtual image is advantageously larger than the sensor area. For example, the virtual image 600 of FIG. 6 was captured and reconstructed according to the just-described procedure. As can be seen, the virtual image 600 is the combination of three overlapping images 611, 612 and 613 each of which is the size of the sensor.

Advantageously, the resulting image 600 has a larger area than the sensor. When a user places a finger on the sensor during an access procedure, alignment errors are overcome by the relatively larger area of the virtual image 600. In other words, the described apparatus and method increases the probability that the portion of the fingerprint placed on the sensor during an access procedure overlaps the enrolled image 600.

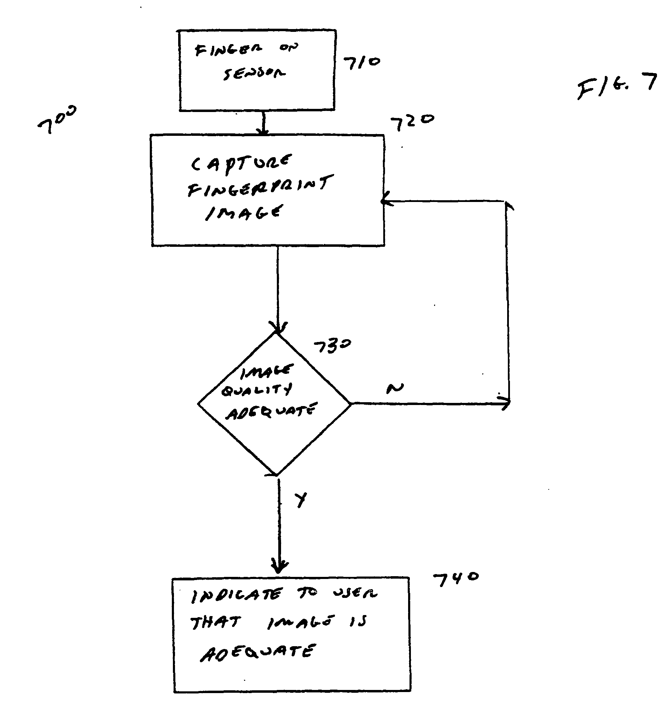

The advantages in overcoming finger placement error with the alignment features of the invention are further enhanced with an image quality indicator, which informs the user when an acceptable image has been captured. A method for providing an image quality indicator is described with reference to the flow chart 700 of FIG 7. In a first step 710 of the procedure, the finger is placed on a sensor enclosed with the previously described apparatus. In a process step 720, the fingerprint image is captured. Then the quality of the image is evaluated in a decision step 730, and it is determined whether the image quality is adequate. If the image is adequate, the user is advised in a process step 740 that the image has been captured. When the image is inadequate, control returns to the process step 720 and the procedure is repeated.

For purposes of this quality indicator feature of a preferred embodiment of the present invention, it is unimportant how an image is captured. Image capturing and storing techniques are well known in the art. Similarly, the image quality may be measured in many ways. For instance, contrast is one known attribute commonly used for evaluating an image; the image is evaluated by how well the intensity range of the image stretches over the maximum intensity range available. Image evaluation is described in W.K. Pratt, "Digital Image Processing," Willey Press, New York, New York. 1978, pp. 307-318. The process step 740, which informs the user that the image quality is adequate, may also be implemented with various methods and apparatus. For instance, the indication may be audible, such as a beep emitted from a speaker, or visual, such as in lighting an LED.

Numerous modifications and alternative embodiments of the invention will be apparent to those skilled in the art in view of the foregoing description. For instance, an enclosure according to the invention is also operable to protect the sensor from dirt, dust or liquids. Similarly, the enclosure and access piece may also comprise a radio frequency shield to protect the sensor from electromagneric energy. Accordingly, this description is to be construed as illustrative only and is for the purpose of teaching those skilled in the art the best mode of carrying out the invention. Details of the structure may be varied substantially and the exclusive use of all modifications which come within the scope of the appended claims are reserved.

## Claims

1. A protective enclosure (100) for a fingerprint sensor (130), comprising:
an access piece (110) positionable between a closed position and at least one open position, said access piece (110) substantially covering the sensor (130) at said closed position and said access piece (110) substantially revealing the sensor (130) at said open position, the enclosure being operable laterally to align the finger on the sensor, and further comprising means for laterally aligning the finger on said sensor in a fingerprint sensing position, wherein said means for laterally aligning comprises:
a first guide (122) being a wall; and,
a second guide (124); and,
means (180) for returning said access piece (110) from said open position to said closed position when said access piece (110) is released;
**characterised by**:
the access piece (110) being electrically conductive,
means for grounding said access piece (110), wherein a finger (120) is substantially grounded upon contacting said access piece (110) said second guide being a wall, said first guide and said second guide being spaced apart by a predetermined width to provide lateral alignment for the finger on said sensor in said fingerprint sensing position.

2. The enclosure of claim 1 wherein said means for returning comprises a spring (180) coupled between said access piece (110) and said enclosure (100), wherein said spring (180) is relaxably coiled when said access piece (110) is at said closed position and said spring is under tension when said access piece (110) is at said open position.

3. The enclosure of claim 1 wherein said access piece (110) is slidable between said closed position and said open position.

4. The enclosure of claim 1 further comprising a means indicative of image quality.

5. The enclosure of claim 4 wherein said means indicative of image quality comprises:
a processing means for capturing an image;
a decision making means for determining when a quality criteria for said image exceeds a predetermined level; and
a means for informing a user when image quality above said predetermined level has been achieved.

6. The enclosure of claim 1 wherein said access piece (110) is positionable at a plurality of positions.

7. The enclosure of claim 1 further comprising a switch (160) disposed relative to said access piece (110), said switch (160) operable to couple the sensor (130) to a power source when said access piece (110) is at said open position and to decouple the sensor (130) from said power source when said access piece (110) is at said closed position.

8. The enclosure of claim 1 wherein the access piece (110) is shaped to form a fingertip contoured area (113) into which a fingertip fits whereby when the access piece (110) is moved to the open position with the fingertip placed in the contoured area (113), the top of the finger (120) extends beyond the sensor (130) and the fingerprint, core is aligned with the sensor (130).

## Patentansprüche

1. Schutzhülle (100) für einen Fingerabdrucksensor (130), die aufweist:
ein Zugriffsstück (110), das zwischen einer geschlossenen Position und zumindest einer offenen Position positionierbar ist, wobei das Zugriffsteil (110) den Sensor (130) bei der geschlossenen Position im Wesentlichen abdeckt und das Zugriffsteil (110) den Sensor (130) in der offenen Position im Wesentlichen freigibt, wobei die Hülle lateral bedienbar ist, um den Finger auf dem Sensor auszurichten, und ferner Mittel aufweist, um den Finger auf dem Sensor lateral in eine Fingerabdruckfühlposition auszurichten, wobei die Mittel zum lateralen Ausrichten aufweisen:
eine erste Führung (122), die eine Wand ist; und
eine zweite Führung (124); und
Mittel (180) um das Zugriffsstück (110) von der offenen Position zu der geschlossenen Position zurückkehren zu lassen, wenn das Zugriffsstück (110) entspannt wird;
**gekennzeichnet durch**
das Zugriffsstück (110) ist elektrisch leitfähig,
Mittel zum Erden des Zugriffsstücks (110), wobei ein Finger (120) im Wesentlichen beim Berühren des Zugriffsstücks (110) geerdet ist,
wobei die zweite Führung eine Wand ist, wobei die erste Führung und die zweite Führung um eine vorbestimmte Breite bzw. Abstand voneinander beabstandet sind, um eine laterale Ausrichtung für den Finger auf dem Sensor in der Fingerabdruckfühlposition zur Verfügung zu stellen.

2. Hülle nach Anspruch 1, wobei das Mittel zum Zurückbringen aufweist, eine Feder (180), die zwischen das Zugriffsstück (110) und die Hülle (100) gekoppelt ist, wobei die Feder (180) entspannbar gewunden ist, wenn das Zugriffsstück (110) in der geschlossenen Position ist, und die Feder unter Spannung ist, wenn das Zugriffsstück (110) in der offenen Position ist.

3. Hülle nach Anspruch 1, wobei das Zugriffsstück (110) zwischen der geschlossenen Position und der offenen Position verschiebbar ist.

4. Hülle nach Anspruch 1, das ferner ein Mittel aufweist, das die Bildqualität anzeigt.

5. Hülle nach Anspruch 4, wobei das Mittel, das die Bildqualität anzeigt aufweist: ein Verarbeitungsmittel zum Einfangen eines Bildes;
eine Entscheidungseinrichtung, um zu bestimmen, wenn ein Qualitätskriterium für das Bild einen vorbestimmten Pegel übersteigt; und
ein Mittel zum Informieren eines Benutzers, wenn die Bildqualität oberhalb des vorbestimmten Pegels erreicht worden ist.

6. Hülle nach Anspruch 1, wobei das Zugriffsstück (110) bei mehreren Positionen positionierbar ist.

7. Hülle nach Anspruch 1, die ferner aufweist, einen Schalter (160), der relativ zu dem Zugriffsstück (110) angeordnet ist, wobei der Schalter (160) betreibbar ist, um den Sensor (130) an eine Leistungsquelle anzukoppeln, wenn das Zugriffsstück (110) in der offenen Position ist, und um den Sensor (130) von der Leistungsquelle abzukoppeln, wenn das Zugriffsstück (110) in der geschlossenen Position ist.

8. Hülle nach Anspruch 1, wobei das Zugriffsstück (110) geformt ist, um eine wie eine Fingerspitze konturierte Fläche (113) zu bilden, in welche eine Fingerspitze passt, wodurch, wenn das Zugriffsstück (110) mit der in der konturierten Fläche (113) angeordneten Fingerspitze in die offene Position bewegt wird, der obere Teil des Fingers (120) über dem Sensor (130) erstreckt ist, und der Kern des Fingerabdrucks ist zu dem Sensor (130) ausgerichtet.

## Revendications

1. Enceinte de protection (100) pour un détecteur d'empreintes digitales (130), comprenant :
une pièce d'accès (110) pouvant être disposée entre une position fermée et au moins une position ouverte, la pièce d'accès (110) recouvrant sensiblement le détecteur (130) à la position fermée et la pièce d'accès (110) découvrant sensiblement le détecteur (130) dans la position ouverte, l'enceinte pouvant être actionnée latéralement pour aligner le doigt sur le détecteur et comprenant en outre un moyen pour aligner latéralement le doigt sur le détecteur dans une position de détection d'empreintes digitales, dans lequel le moyen d'alignement latéral comprend :
un premier guide (122) formé d'une paroi ;
un second guide (124) ; et
des moyens de rappel (180) de la pièce d'accès (110) de la position ouverte à la position fermée quand la pièce d'accès (110) est libérée ;
**caractérisé par :**
**le fait que** la pièce d'accès (110) est électriquement conductrice ;
des moyens pour mettre à la masse la pièce d'accès (110) quand un doigt (120) est sensiblement à la masse par suite du contact avec la pièce d'accès (110) ; et
**le fait que** le second guide est une paroi, le premier et le second guide étant espacés d'une largeur prédéterminée pour assurer un alignement latéral du doigt sur le détecteur dans la position de prise d'empreintes.

2. Enceinte selon la revendication 1, dans laquelle les moyens de rappel comprennent un ressort (180) couplé entre la pièce d'accès (110) et l'enceinte (100), dans lequel le ressort (180) est bobiné de façon libérable quand la pièce d'accès (110) est dans la position fermée et le ressort est sous tension quand la pièce d'accès (110) est en position ouverte.

3. Enceinte selon la revendication 1, dans laquelle la pièce d'accès (110) peut coulisser entre la position fermée et la position ouverte.

4. Enceinte selon la revendication 1, comprenant en outre un moyen indicatif d'une qualité d'image.

5. Enceinte selon la revendication 4, dans laquelle ledit moyen indicatif de la qualité d'image comprend :
un moyen de traitement pour capter une image ;
un moyen de prise de décision pour déterminer quand un critère de qualité d'image dépasse un niveau prédéterminé ; et
un moyen pour informer un utilisateur quand la qualité d'image a atteint un niveau supérieur au niveau prédéterminé.

6. Enceinte selon la revendication 1, dans laquelle la pièce d'accès (110) peut être positionnée dans plusieurs positions.

7. Enceinte selon la revendication 1, comprenant un commutateur (160) disposé par rapport à la pièce d'accès (110), le commutateur (160) étant actionnable pour coupler le détecteur (130) à une source d'alimentation quand la pièce d'accès (110) est en position ouverte et pour découpler le détecteur (130) de la source d'alimentation quand la pièce d'accès (110) est en position fermée.

8. Enceinte selon la revendication 1, dans laquelle la pièce d'accès (110) a une forme propre à se conformer à la surface d'un doigt (113) dans laquelle une extrémité de doigt s'adapte, d'où il résulte que, quand la pièce d'accès (110) est déplacée vers la position ouverte, l'extrémité du doigt étant placée dans la zone conformée (113), le sommet du doigt (120) s'étend au-delà du détecteur (130) et le coeur de l'empreinte digitale est aligné avec le détecteur (130).
